(19) Europäisches Patentamt
European Patent Office
Office européen des brevets

(11) Numéro de publication: 0 099 802
B1

(12) # FASCICULE DE BREVET EUROPÉEN

(45) Date de publication du fascicule du brevet: **04.02.87**

(51) Int. Cl.⁴: **C 07 D 495/04,** A 61 K 31/435 // (C07D495/04, 333:00, 221:00)

(21) Numéro de dépôt: **83401382.3**

(22) Date de dépôt: **05.07.83**

(54) **Nouveaux dérivés de la thiéno-(3,2-c) pyridine, leur procédé de préparation et leur application thérapeutique.**

(30) Priorité: **13.07.82 FR 8212599**

(43) Date de publication de la demande: **01.02.84 Bulletin 84/5**

(45) Mention de la délivrance du brevet: **04.02.87 Bulletin 87/6**

(84) Etats contractants désignés: **AT BE CH DE FR GB IT LI LU NL SE**

(56) Documents cité:
**FR-A-1 312 412**
**FR-A-2 215 948**

**Le dossier contient des informations techniques présentées postérieurement au dépôt de la demande et ne figurant pas dans le présent fascicule.**

(73) Titulaire: **SANOFI, Société dite:, 40, Avenue George V, F-75008 Paris (FR)**

(72) Inventeur: **Aubert, Daniel, Chemin d'Encrabe, F-31170 Plaisance du Touch (FR)**
Inventeur: **Ferrand, Claude, 9, rue de l'Ancienne Batterie, F-31520 Ramonville Saint- Agne (FR)**
Inventeur: **Maffrand, Jean- Pierre, 5, rue du Corps Franc- Pommiès, F-31120 Portet/Garonne (FR)**

(74) Mandataire: **Polus, Camille, c/o Cabinet Lavoix 2, Place d'Estienne d'Orves, F-75441 Paris Cedex 09 (FR)**

EP 0 099 802 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1987

**Description**

L'invention est relative à de nouvelles thiéno [3,2-c] pyridines, à un procédé pour les préparer et à leurs applications en thérapeutique.

On connaît des dérivés tétrahydro-4,5,6,7 thiéno [3,2 c] pyridine portant notamment sur l'atome d'azote un groupe benzyle pouvant être substitué sur le noyau phényle selon FR-A-2 215 948, utiles comme médicaments.

L'invention propose de nouveaux dérivés ayant la même structure de base, mais en plus un groupe carboxy, alcoxycarbonyle ou carboxamido en position α sur le groupe benzyle. Cette modification de structure a eu comme résultat surprenant une nette amélioration de l'activité inhibitrice de l'agrégation plaquettaire, permettant de prévoir l'utilisation de doses thérapeutiques inférieures tout en diminuant nettement le niveau de toxicité de la plupart des composés.

Les nouveaux dérivés suivant l'invention répondent à la formule générale I

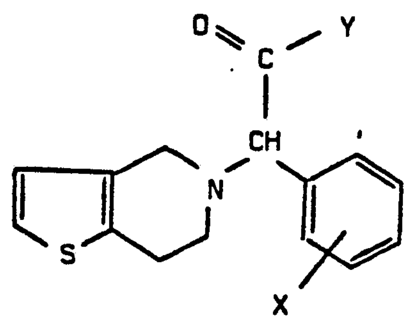

(I)

dans laquelle Y peut représenter l'hydroxyle OH ou le groupe OR dans lequel R est un radical alcoyle inférieur droit ou ramifié, ou bien Y représente un groupe

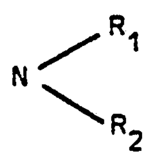

dans lequel $R_1$ et $R_2$ sont chacun indépendamment l'un de l'autre l'hydrogène ou un groupe alcoyle inférieur droit ou ramifié: ou bien $R_1$ et $R_2$ forment ensemble et avec l'atome d'azote auquel ils sont rattachés un groupe pyrolidino, morplidino, pipéridino ou benzyl-4 pipéramino, et X représente l'hydrogène, un halogène ou un radical alcoyle inférieur.

Ces composés comportant un carbone assymétrique peuvent exister sous la forme de 2 énantiomères. L'invention concerne aussi bien chacun des énantiomères que leur mélange.

L'invention comprend aussi les sels d'addition avec les acides minéraux ou organiques pharmaceutiquement acceptables lorsque Y représente les groupes OR ou R

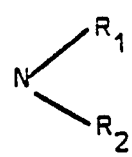

ou avec les bases minérales lorsque Y représente OH.

Par radical alcoyle inférieur, on entend une chaîne hydrocarbonée saturée en $C_1$-$C_4$.

L'invention a également pour objet un procédé de préparation des composés de formule générale I ci-dessus caractérisé en ce que l'on prépare les esters de l'invention dans lesquels Y représente un groupe OR tel qu'il est défini ci-dessus par condensation de la tétrahydro-4,5,6,7 thiéno [3.2-c] pyridine, de formule II

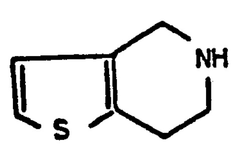

(II)

avec un α-chlorophényl-acétate de formule générale III

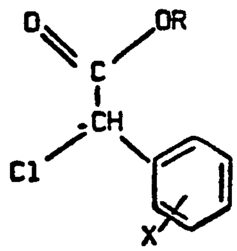

(III)

dans lequel R et X prennent les valeurs définies ci-dessus. Par saponification, on obtient les acides de formule générale I dans lesquels Y représente l'hydroxyle OH.

Pour préparer les amides, dans lesquels Y représente un groupe

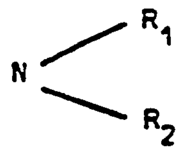

dans lequel $R_1$ et $R_2$ ont les valeurs définies ci-dessus ou même certains esters de formule générale I l'acide formule générale I (R = OH) est mis à réagir, éventuellement après activation soit avec l'amine

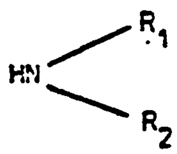

soit avec l'alcool R-OH.

Les esters α-halogénés de formule générale III sont préparés selon des méthodes connues (E.L. ELIEL, M.T. FISK et T. PROSSER. Organic Syntheses, Coll. Vol. IV, J. WILEY et SONS, Inc. New-York, 1963, p. 159).

S'il est tout-à-fait possible d'obtenir tous les esters de formule générale I par la réaction entre les composés des formules (II) et (III). il est préférable, sur le plan économique, de préparer certains esters supérieurs de formule générale I à partir de l'acide de formule générale I et de l'alcool R-OH.

La condensation de la tétrahydro-thiéno-pyridine avec l'ester de formule générale III est effectuée en présence d'un carbonate de métal alcalin, tel que par exemple, le carbonate de potassium, dans un solvant inerte tel que le diméthylformamide, le tétrahydrofuranne ou le diméthoxy-1,2 éthane, à des températures comprises entre 60° C et le point d'ébullition du solvant,

La saponification de l'ester de formule générale I dans lequel R est méthyle ou éthyle est effectuée par un hydroxyde de métal alcalin, tel que la soude ou la potasse, au sein d'un solvant hydro-alcoolique, à des températures comprises entre la température ambiante et le point d'ébullition du solvant.

L'activation de l'acide de formule générale I peut être obtenue par traitement à l'aide du chloroformiate d'éthyle en présence d'un léger excès de triéthylamine, à des températures comprises entre -5° C et 0° C dans un solvant inerte tel que le chloroforme, le diméthoxy-1,2 éthane ou le tétrahydrofuranne.

Il se forme un anhydride mixte de formule générale IV

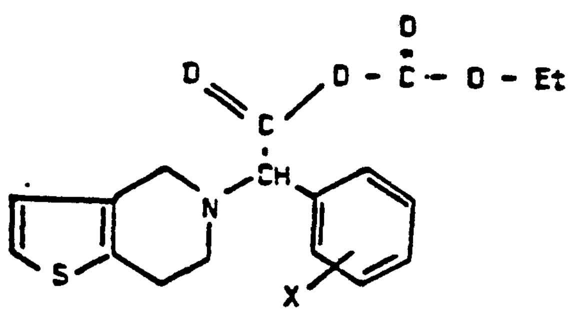

(IV)

dont le traitement, in situ, par un léger excès soit de l'alcool, soit de l'amine, à des températures comprises entre 10° C et la température ambiante, conduit respectivement aux esters ou aux amides de formule générale I.

L'activation de l'acide de formule générale I peut aussi être obtenue de différentes manières: ainsi on a aussi préparé les amides de formule générale I en condensant l'acide I avec l'amine

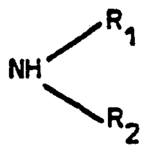

en présence de dicyclohexycarbodiimide en solution dans le dichloro 1,2-éthane.

Quant aux esters de formule générale I, ils peuvent être aussi obtenus de façon classique en condensant l'acide et l'alcool ROH correspondants en présence ce gaz chlorhydrique ou de chlorure de thionyle.

Les exemples non limitatifs suivants sont donnés à titre d'illustration de l'invention.

**Exemple 1**

α-(tétrahydro-4,5,6,7 thiéno [3,2-c] pyridyl-5) o.chlorophénylacétate de méthyle (R = $-CH_3$;X = 2-Cl), dérivé n° 1.

A une solution de 20 g (0,144 mole) de tétrahydro-4,5,6,7 thiéno [3.2-c] pyridine dans 200 ml de diméthylformamide on ajoute 31,47 g (0,144 mole) de chloro-2, o.chloro phénylacétate de méthyle et 19,82 g (0,144 mole) de carbonate de potassium, puis on chauffe à 90°C pendant 4 heures. Le milieu réactionnel est refroidi à température ambiante, les sels minéraux sont filtrés et le solvant est évaporé. Le résidu est repris à l'eau, puis extrait à l'éther éthylique. Les extraits éthérés sont lavés à l'eau. séchés sur sulfate de sodium et après évaporation on recueille une huile jaune, que l'on purifie par l'intermédiaire de son chlorhydrate: cristaux blancs: F = 130-140°C (acétate d'éthyle, isopropanol),

rendement: 45%.

**Exemple 2**

α-(tétrahydro-4,5,6,7 thiéno [3,2-c] pyridyl-5) phénylacétate de méthyle (R = $-CH_3$:X=H), dérivé n° 2.

Ce composé est préparé selon le mode opératoire décrit dans l'exemple 1 par alcoylation de la tétrahydro-4,5,6,7 thiéno [3,2-c] pyridine avec le chloro-2 phénylacétate de méthyle.

Chlorhydrate: cristaux blancs: F = 200°C (éthanol).

rendement: 50 %.

**Exemple 3**

α-(tétrahydro-4,5,6,7 thiéno [3,2-c] pyridyl-5) o.fluoro phénylacétate de méthyle (R = $-CH_3$; X = 2-F), dérivé n° 3.

Ce composé est préparé selon le mode opératoire décrit dans l'exemple 1 par alcoylation de la tétrahydro-4,5, 6,7 thiéno [3,2-c] pyridine avec le chloro-2 o.fluoro phénylacétate de méthyle.

Chlorhydrate: cristaux blancs: F pâteux =100°C.

rendement: 76,5 %.

**Exemple 4**

α-(tétrahydro-4,5,6,7 thiéno [3,2-c] pyridyl-5) o.méthyl phénylacétate d'éthyle. (R = $-CH_2-CH_3$: X = 2-$CH_3$), dérive n° 4.

Ce composé est préparé selon le mode opératoire décrit dans l'exemple 1 par alcoylation de la tétrahydro-4,5,6,7 thiéno [3,2-c] pyridine avec le chloro-2 o.méthyl phénylacétate d'éthyle.

Hémisulfate; cristaux blancs: F = 188-190°C (isopropanol).

rendement: 54 %.

**Exemple 5**

Acide α-(tétrahydro-4,5,6,7 thiéno [3,2-c] pyridyl-5) o.chloro pphénylacétique. (Y = OH ; X = 2-Cl), dérivé n° 5.

On chauffe à reflux 2 heures 30 un mélange de 157,9 g d'α-(tétrahydro-4,5,6,7 thiéno [3,2-c] pyridyl-5) o.chloro phénylacétate d'éthyle et de 100 ml de lessive de soude à 30% dans 600ml d'éthanol. Après évaporation de l'éthanol, le milieu est acidifié à l'acide acétique glacial et extrait au chlorure de méthylène. La phase organique est lavée à l'eau, séchée sur sulfate de sodium puis évaporée. Après recristallisation dans l'eau, le produit est isolé sous forme monohydraté.
Cristaux blancs: F pâteux = 125°C (eau).
rendement: 46 %.

**Exemple 6**

Acide α-(tétrahydro-4,5,6,7 thiéno [3,2-c] pyridyl-5) phénylacétique. (Y = OH ; X = H), dérivé n° 6.
Ce composé est préparé selon le mode opératoire décrit dans l'exemple 5 par saponification d'α-(tétrahydro-4,5,6,7 thiéno [3,2-c] pyridyl-5) phénylacétate d'éthyle. Le produit est purifié par l'intermédiaire de son sel sodique.
Cristaux blancs: F = 210-215°C (éthanol, méthanol).
rendement: 74 %.

**Exemple 7**

α-(tétrahydro-4,5,6,7 thiéno [3,2-c] pyridyl-5) a chloro phénylacétate de n-propyle. (R = $-CH_2-CH_2-CH_3$ ; X = 2-Cl), dérivé n° 7.
On fait barboter pendant 12 heures un courant de gaz chlorhydrique dans une solution de 10 g (0,0306 mole) d'acide α-(tétrahydro-4,5,6,7 thiéno [32-c] pyridyl-5) o.chloro phénylacétique monohydraté (exemple 5) dans 100ml de n-propanol que l'on porte au reflux. Le milieu est évaporé et le résidu est repris à l'eau, basifié au bicarbonate de sodium et extrait à l'éther éthylique. Les extraits éthérés sont lavés à l'eau, séchés sur sulfate de sodium, et après évaporation on recueille une huile jaune que l'on purifie par l'intermédiaire de son hémisulfate.
cristaux blancs: F = 145°C (brut).
rendement: 78%.

**Exemple 8**

α-(tétrahydro-4,5,6,7 thiéno [3,2-c] pyridyl-5) o.chloro phénylacétate de n-butyle. (R $-CH_2-CH_2-CH_2-CH_3$ ; X = 2-Cl) dérivé n° 8.
Ce composé est préparé selon le mode opératoire décrit dans l'exemple 7 par estérification de l'acide α-(tétrahydro-4,5,6,7 thiéno [3,2-c] pyridyl-5) o.chloro phénylacétique monohydraté (exemple 5) avec le n-butanol. Purification par l'intermédiaire de l'hémisulfate ; cristaux blancs: F = 155°C. Rendement: 73,5 %.

**Exemple 9**

α-(tétrahydro-4,5,6,7 thiéno [3,2-c] pyridyl-5) o.chloro phénylacétate d'isopropyle. (R =

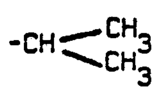

; X = 2-Cl), dérivé n° 9.
A une suspension de 1 g (0,0031 mole) d'acide α-(tétrahydro-4,5,6,7 thiéno [3,2-c] pyridyl-5) o.chloro phénylacétique monohydraté (exemple 5) dans 20 ml d'isopropanol refroidie à -10°C. on ajoute goutte à goutte 2 ml de chlorure de thionyle, puis le milieu réactionnel est chauffé 6 heures à reflux. Après évaporation, le résidu est repris à l'eau, basifié au bicarbonate de sodium et extrait au chlorure de méthylène. La phase organique est lavée à l'eau, séchée sur sulfate de sodium et évaporée. On recueille une résine incolore qui est purifiée par l'intermédiaire de son hémisulfate.
Cristaux blancs: F pâteux = 140-150°C.
rendement: 44 %.

**Exemple 10**

α-(tétrahydro-4,5,6,7 thiéno [3,2-c] pyridyl-5) o.chloro phénylacétate d'éthyle. (R = $CH_2$-$CH_3$ ; X = 2-Cl), dérivé n° 10.

A une solution de 75 g (0.046 mole) d'acide α-(tétrahydro-4,5,6,7 thiéno [3,2-c] pyridyl-5) o.chloro phénylacétique monohydraté (exemple 5) et 7,12 ml (0.051 mole) de triéthylamine dans 150 ml de chloroforme, refroidie à une température comprise entre -5° et 0°C on ajoutegoutte à goutte 4,86 ml (0,051 mole) de chloroformiate d'éthyle. L'introduction terminée on laisse revenir à température ambiante et agite 1/2 heure. Le milieu réactionnel est ensuite refroidi à une température voisine de 10°C et 30 ml d'éthanol sont ajoutés goutte à goutte. Le mélange réactionnel est agité à température ambiante une nuit, puis lavé à l'eau. La phase organique, séchée sur sulfate de sodium est évaporée pour donner une huile incolore que l'on purifie par l'intermédiaire de son bromhydrate.

Cristaux blancs: F déc. = 180°C.

rendement: 94 %.

**Exemple 11**

N,N-diméthyl [α-(tétrahydro-4,5,6,7 thiéno [3,2-c] pyridyl-5)] o.chloro phénylacétamide.

( Y = 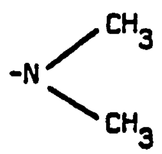

; X = 2-Cl), dérivé n° 11.

A une solution de 30 g (0,092 mole) d'acide α-(tétrahydro-4,5,6,7 thiéno [3,2-c] pyridyl-5) o.chloro phénylacétique monohydraté (exemple 5) et de 14,24 ml (0,102 mole) de triéthylamine dans 300 ml de chloroforme, refroidie à une température comprise entre -5 et 0°C. on ajoute goutte à goutte 9,72 ml (0,102 mole) de chloroformiate d'éthyle. L'introduction terminée on laisse revenir à température ambiante et on agite le milieu réactionnel pendant une demi heure. Ce dernier est ensuite refroidi à une température voisine de 10°C puis on ajoute goutte à goutte 4,57 ml (0,102 mole) de diméthylamine dans 60 ml de chloroforme et on agite à la température ambiante une nuit.

On ajoute de l'eau, décante, et la phase organique est séchée sur sulfate de sodium et évaporée. On recueille une résine incolore qui cristallise.

Cristaux blancs: F = 95-100°C (éther isopropylique).

rendement: 49%.

**Exemple 12**

[(chloro-2 phényl) (tétrahydro-4,5,6,7 thiéno [3,2-c] pyridyl-5) acétyl]-1 pyrrolidine.

(Y = 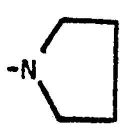

; X = 2-Cl), dérivé n° 12.

Ce composé est préparé selon le mode opératoire décrit dans l'exemple 11 par condensation de l'acide α-(tétrahydro-4,5,6,7 thiéno [2,3-c] pyridyl-5) o.chloro phénylacétique monohydrate (exemple 5) avec la pyrrolidine.

Cristaux blancs: F = 130°C (éther isopropylique).

rendement: 61,5%.

**Exemple 13**

[(chloro-2 phényl) (tétrahydro-4,5,6,7 thiéno [3,2-c] pyridyl-5) acétyl]-1 morpholine.

(Y = 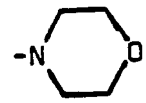

; X = 2-Cl), dérivé n° 13

A une solution de 10 g (0,031 mole) d'acide α-(tétrahydro-4,5,6,7 thiéno [3,2-c] pyridyl-5) o. chloro phénylacétique monohydrate (exemple 5) et de 13,3 g (0,064 mole) de dicyclohexylcarbodiimide dans 100 ml de dichloro-1,2 éthane on ajoute 2,67 g (0,031 mole) de morpholine et on agite à température ambiante pendant une nuit. Le milieu est évaporé puis repris à l'acide chlorhydrique 2N et l'éther éthylique. Après filtration de la dicyclohexylurée formée, le filtrat est décanté et la phase aqueuse est basifiée à la soude 2N puis extraite au chlorure de méthylène. La phase organique est lavée à l'eau, séchée sur sulfate de sodium et évaporée pour donner une résine jaune que l'on purifie par l'intermédiaire de son chlorhydrate hémihydrate.

Cristaux blancs: F = 215-225° C (isopropanol).

rendement: 71 %.

**Exemple 14**

[(chloro-2 phényl) (tétrahydro-4,5,6,7 thiéno [3,2-c] pyridyl-5) acétyl]-1 pipéridine.

(Y = 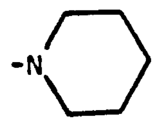

; X = 2-Cl), dérivé n° 14.

Ce composé est préparé selon le mode opératoire décrit dans l'exemple 13 par condensation de l'acide α-(tétrahydro-4,5,6,7 thiéno [3,2-c] pyridyl-5) o. chloro phénylacétique monohydraté (exemple 5) avec la pipéridine.

Cristaux blancs: F = 139° C (isopropanol).

rendement: 51,5%.

Selon le procédé décrit dans l'exemple 11 on a préparé les composés suivants:

[α-(tétrahydro-4,5,6,7 thiéno [3,2-c] pyridyl-5)] o.chloro phénylacétamide. (Y = $-NH_2$ ; X = 2-Cl), dérivé n° 15.

Cristaux blancs: F = 126-128° C (éther isopropylique-isopropanol). rendement: 45%.

- benzyl-4[(chloro-2 phényl) (tétrahydro-4,5,6,7 thiéno [3,2-c] pyridyl-5) acétyl]-1 pipérazine.

(Y = 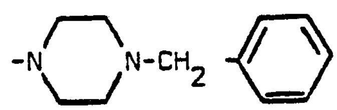

; X = 2-Cl), dérivé n° 16.

oxalate: cristaux blancs: F = 178° C (éthanol).

rendement: 82,5%.

- N,N-diméthyl [α-(tétrahydro-4,5,6,7 thiéno [3,2-c] pyridyl-5)] o.fluoro phénylacétamide

(Y = 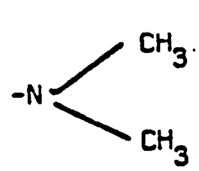

; X = 2-F), dérivé n° 17.

Poudre légèrement jaune: F = 125° C (éther isopropylique - isopropanol),

rendement: 41 %.

- N-méthyl [α-(tétrahydro-4,5,6,7 thiéno [3,2-c] pyridyl-5)] o.chloro phénylacétamide. (Y = $NH-CH_3$ ; X = 2-Cl), - dérivé n° 18.

Cristaux blancs: F= 137° C (isopropanol).
rendement: 85,5 %.

- N-butyl [α-(tétrahydro-4,5,6,7 thiéno [3,2-c] pyridyl-5)] o.chloro phénylacétamide. (Y= $-NH-(CH_2)_3-CH_3$ ; X = 2-Cl)-dérivé n° 19. Cristaux blancs: F = 101°C (éther isopropylique).
rendement: 65 %.

- N,N-diméthyl [α-(tétrahydro-4,5,6,7 thiéno [3,2-c] pyridyl-5)] phénylacétamide.

(Y = 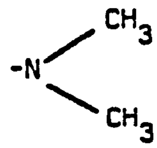

; X = H), dérivé n° 20.
Cristaux blancs: F = 138° C (éther isopropylique).
rendement: 39%.

- N,N-diméthyl [α-(tétrahydro-4,5,6,7 thiéno [3,2-c] pyridyl-5)] o.méthyl phénylacétamide.

(Y = 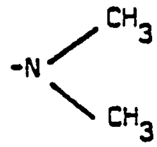

; X = $2-CH_3$) dérivé n° 21.
Cristaux blancs: F = 119° C (hexane).
rendement: 15%.

Les résultats pharmacologiques et toxicologiques qui sont rapportés ci-dessous mettent en évidence les propriétés des dérivés de l'invention tant sur le plan de la toxicité et ce la tolérance, que sur le plan de leurs activités, notamment inhibitrice ce l'agrégation plaquettaire et anti-thrombotique.

L'invention a donc encore pour objet un médicament présentant en particulier des activités inhibitrice de l'agrégation plaquettaire et anti-thrombotique, caractérisé en ce qu'il contient, à titre de principe actif, un dérivé de formule générale I ou un sel d'addition avec un acide minéral ou organique pharmaceutiquement acceptable lorsque Y représente les groupes OR ou

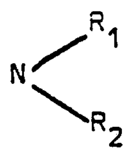

et avec une base minérale lorsque Y est OH.

## Etude toxicologique

Les composés de l'invention bénéficient d'une excellente tolérance et d'une faible toxicité.

En outre, les essais effectués sur les toxicités aiguë, chronique, sub-chronique et retardée chez différentes espèces animales n'ont pas permis de mettre en évidence une quelconque réaction locale ou générale, perturbation ou anomalie dans les examens biochimiques macroscopiques ou microscopiques effectués durant cette expérimentation.

## Etude pharmacologique

### 1°) Action inhibitrice de l'agrégation plaquettaire

Cette expérimentation est effectuée sur le rat qui a reçu, pendant 3 jours, par la voie orale, le composé à tester aux moments -48 h. -24 h et -2 h, en suspension dans la gomme arabique. Au moment 0 h, on prélève 4 ml de sang selon la technique de Renaud à la veine jugulaire de l'animal anesthésié. C'est ce sang citraté qui est utilisé dans les mesures d'agrégation.

a) <u>mesure de l'agrégation plaquettaire à l'A.D.P.</u>

2 ml de sang citraté sont rapidement versés dans un petit becher placé sur un agitateur magnétique et pourvu d'une barre aimantée. Après quelques secondes d'agitation, on introduit dans le becher 0.4 ml d'une solution contenant 0.66 μg d'adénosine-diphosphate (A.D.P.) par ml. Après 90 secondes d'agitation, on procède à deux prélèvements de 0,5 ml de sang:

- la premier est mélange avec 0,5 ml d'une solution EDTA-formol,
- le deuxième est mélangé avec 0,5 ml d'une solution EDTA seulement.

L'addition d'EDTA-formol a pour but de stabiliser le sang et donc de fixer l'agrégation tandis que l'EDTA provoque au contraire la désegrégation de tous les amas plaquettaines.

Après un repos de 10 minutes et une centrifugation des 2 mélanges à vitesse lente pendant 5 minutes, afin de séparer les globules rouges, le plasma riche en plaquettes (PRP) surnageant est prélevé, dilué et numéré en plaquettes.

L'intensité de l'agrégation est déterminée par le rapport

$$\frac{\text{nombre de plaquettes dans EDTA-formol}}{\text{nombre de plaquettes dans EDTA}} \times 100 = \text{pourcentage de plaquettes non agrégées.}$$

Le produit à tester est d'autant plus inhibiteur de l'agrégation plaquettaire que le rapport se rappoche de 100.

Les résultats que expriment le pourcentage moyen de plaquettes non agrégées dans les lots de 5 rats (traités et témoins) sont rassemblés dans le tableau I.

**Tableau I**: Test a l'ADP

| PRODUIT | DOSE mg/kg | VOIE | RESULTAT |
|---|---|---|---|
| Témoin | | P.O. | 16 ± 4 |
| dérivé n° 1 | 3 x 25 | - | 94 ± 3 |
| Témoin | | - | 20 ± 11 |
| dérivé n° 1 | 3 x 5 | - | 82 ± 11 |
| Témoin | | - | 23 ± 15 |
| dérivé n° 1 | 3 x 2,5 | - | 56 ± 17 |
| Témoin | | - | 8 ± 0 |
| dérivé n° 10 | 3 x 25 | - | 66 ± 2 |
| dérivé n° 10 | 3 x 12,5 | - | 49 ± 11 |
| Témoin | | - | 8 ± 1 |
| dérivé n° 10 | 3 x 10 | - | 24 ± 5 |
| Témoin | | - | 11 ± 0 |
| dérivé n° 9 | 3 x 60 | - | 65 ± 7 |
| Témoin | | - | 13 ± 3 |
| dérivé n° 4 | 3 x 100 | - | 89 ± 1 |
| Témoin | | - | 3 ± 1 |
| dérivé n° 4 | 3 x 100 | - | 89 ± 4 |
| Témoin | | - | 4 ± 0 |
| dérivé n° 2 | 3 x 100 | - | 72 ± 12 |
| Témoin | | - | 4 ± 0 |
| dérivé n° 12 | 3 x 100 | - | 27 ± 5 |
| Témoin | | - | 2 ± 0 |
| dérivé n° 17 | 3 x 100 | - | 11 ± 3 |
| dérivé n° 20 | 3 x 100 | - | 4 ± 1 |
| Témoin | | - | 18 ± 1 |
| dérivé n° 6 | 3 x 100 | - | 22 ± 4 |

b) <u>mesure de l'agrégation plaquettaire au collagène</u>

1,5 ml de sang citraté est additionné de 0.10 ml d'une solution contenant 10 µg de collagène par ml. Le milieu étant maintenu en agitation, le comptage des plaquettes est effectué sans interruption.

La diminution du nombre de plaquettes libres, en fonction du temps est suivie en continu et permet de tracer une courbe dont la pente donne la vitesse initiale de l'agrégation.

Les résultats, représentant les moyennes établies à l'intérieur de chaque lot de 5 rats (traitès et témoins). sont rassemblés dans le tableau II.

**Tableau II**: Test au collagène

| PRODUIT | DOSE mg/kg | VOIE | RESULTAT |
|---|---|---|---|
| Témoin | | P.O. | 3,12 ± 0,47 |
| dérivé n° 1 | 3 x 25 | - | 0,14 ± 0,03 |
| Témoin | | - | 2,17 ± 0,64 |
| dérivé n° 1 | 3 x 5 | - | 0,19 ± 0,04 |
| Témoin | | - | 5,00 ± 1,02 |
| dérivé n° 1 | 3 x 2,5 | - | 0,60 ± 0,20 |
| Témoin | | - | 3,92 ± 0,63 |
| dérivé n° 10 | 3 x 25 | - | 0,16 ± 0,07 |
| dérivé n° 10 | 3 x 12, 5 | - | 0,54 ± 0,12 |
| Témoin | | - | 2,00 ± 0,35 |
| dérivé n° 7 | 3 x 100 | - | 0,66 ± 0,18 |
| dérivé n° 8 | 3 x 100 | - | 0,86 ± 0,18 |
| Témoin | | - | 2,25 ± 0,32 |
| dérivé n° 9 | 3 x 60 | - | 0,11 ± 0,01 |
| Témoin | | - | 3,41 ± 0,55 |
| dérivé n° 4 | 3 x 100 | - | 0,12 ± 0,02 |
| Témoin | | - | 4,73 ± 0,55 |
| dérivé n° 4 | 3 x 100 | - | 0,30 ± 0,02 |
| Témoin | | - | 5,00 ± 1,06 |
| dérivé n° 2 | 3 x 100 | - | 0,51 ± 0,16 |
| Témoin | | - | 2,25 ± 0,32 |
| dérivé n° 11 | 3 x 100 | - | 0,83 ± 0,02 |
| Témoin | | - | 2,77 ± 0,32 |
| dérivé n° 14 | 3 x 100 | - | 1,89 ± 0,13 |
| Témoin | | - | 3,99 ± 0,40 |
| dérivé n° 17 | 3 x 100 | - | 2,22 ± 0,10 |
| Témoin | | - | 5,01 ± 0,79 |
| dérivé n° 21 | 3 x 100 | - | 3,04 ± 0,22 |
| Témoin | | - | 11,35 ± 1,01 |
| dérivé n° 18 | 3 x 100 | - | 10,82 ± 0,81 |

c) <u>mesure du temps de saignement</u>

L'étude de l'activité inhibitrice do l'agrégation plaquettaire a porté aussi sur l'action du composé, de l'invention vis-à-vis du temps de saignement.

La méthode utilisée est une adaptation de la technique de L. STELLA. M.B. DONATI et G. de GAETANO. Thromb. Res., 1975, 7, 709-716.

L'expérimentation est effectué sur le rat qui a reçu, 65 heures, 41 heures et 17 heures auparavant un traitement per os du composé à tester en suspension dans 10 ml/kg d'une solution aqueuse de gomme arabique à 5%. Après anesthésie au pentobarbital, la queue du rat est sectionnée à 5 mm de l'extrémité. Le sang est soigneusement épongé toutes les 15 secondes en prenant soin de ne pas toucher la plaie.

L'hemostase est atteinte lorsqu'il y a arrêt du saignement pendant une minute.

Les résultats représentant les temps moyens de saignement, exprimés en secondes, établis à l'intérieur de chaque lot de 5 rats (témoins et traités), sont rassemblés dans le tableau III. Les temps supérieurs à 1200 secondes (20 minutes) ne sont plus comptés.

**Tableau III**: Temps de saignement

| PRODUIT | DOSE mg/kg | VOIE | RESULTAT |
|---|---|---|---|
| Témoin | | P.O. | 600 |
| dérivé n° 10 | 3 x 200 | - | > 1 200 |
| dérivé n° 2 | 3 x 200 | - | > 1 200 |
| Témoin | | - | 420 |
| dérivé n° 1 | 3 x 25 | - | > 1 200 |
| dérivé n° 1 | 3 x 5 | - | 1 080 |
| Témoin | | - | 435 |
| dérivé n° 1 | 3 x 12,5 | - | > 1 200 |
| Témoin | | - | 780 |
| dérivé n° 3 | 3 x 200 | - | > 1 200 |
| Témoin | | - | 600 |
| dérivé n° 18 | 3 x 200 | - | > 1 200 |
| Témoin | | - | 600 |
| dérivé n° 12 | 3 x 200 | - | > 1 200 |

2°) <u>Activité anti-thrombotique</u>

Cette activité a été étudiée selon la méthode de la thrombose expérimentale sur fil de soie.

Le principe de cette étude est une adaptation de la méthode de la thrombose expérimentale par circulation extra-corporelle, décrite par TERUHIKO UMETSU et KAZUKO SANAI (TROMB. HAEMOST., 39, 1, 1978).

Sur le rat anesthésié par une injection intra-péritonéale de pentobarbital, la jugulaire gauche et la carotide externe droite sont mises à nu.

Le shunt artério-veineux est constitué par un catheter central et deux catheters latéraux: un fil de soie naturelle blanche est introduit dans la partie centrale et la circulation rétablie pendant 20 minutes. Après arrêt de la circulation par clampage, le fil est retiré doucement et pesé immédiatement. Le poids moyen d'un fil de soie humide ayant été déterminé au préalable, on obtient par différence, le poids du thrombus.

Le traitement est pratiqué 46 heures, 24 heures et 2 heures avant le début de la circulation sanguine dans le shunt par l'administration orale du composé à tester en suspension dans 10 ml/hg de gomme arabique à 5%. le témoin ne recevant que la solution de gomme arabique à 5%.

Les résultats représantant le poids du thrombus en mg son rassemblés dans le tableau IV.

**Tableau IV**: Activité anti-thrombotique

| PRODUIT | DOSE mg/kg | VOIE | POIDS DU THROMBUS EN mg | VARIATION EN POURCENTAGE |
|---|---|---|---|---|
| Témoin | | P.O. | 38,56 ± 2,42 | |
| dérivé n° 8 | 3 x 200 | - | 29,99 ± 3,05 | -22 |
| Témoin | | - | 42,65 ± 3,30 | |
| dérivé n° 3 | 3 x 200 | - | 2,60 ± 0,24 | -94 |
| Témoin | | - | 36,24 ± 2,05 | |
| dérivé n° 1 | 3 x 25 | - | 6,56 ± 0,51 | -82 |
| dérivé n° 1 | 3 x 12,5 | - | 15,98 ± 1,81 | -56 |
| Témoin | | - | 40,86 ± 2,02 | |
| dérivé n° 1 | 3 x 5 | - | 27,7 ± 2,82 | -32 |
| Témoin | | - | 40,68 ± 1,74 | |
| dérivé n° 2 | 3 x 200 | - | 8,42 ± 3,28 | -79 |
| dérivé n° 10 | 3 x 200 | - | 5,89 ± 0,99 | -86 |
| Témoin | | - | 35,76 ± 1,76 | |
| dérivé n° 11 | 3 x 200 | - | 21,38 ± 2,92 | -40 |

Les études toxicologique et pharmacologique qui viennent d'être rapportées ont mis en évidence la faible toxicité des composés de l'invention, ainsi que leur excellente tolérance et leurs intéressantes propriétés inhibitrice de l'agrégation plaquettaire et anti-thrombotique qui les rendent très utiles en thérapeutique humaine et vétérinaire.

Le médicament de l'invention peut être présenté pour l'administration orale sous forme de comprimés, comprimés dragéifiés, capsules, gouttes, granulés ou sirop. Il peut aussi être présenté pour l'administration rectale, sous forme de suppositoires et, pour administration parentérale, sous forme de soluté injectable.

Chaque dose unitaire contient, avantageusement de 0,005 g à 0,250 g d'un dérivé de l'invention, les doses administrables journellement pouvant varier de 0,005 g à 1,00 g de principe actif en fonction de l'âge de malade et de la sévérité de l'affection traitée.

On donnera ci-après, à titre d'exemples non limitatifs, quelques formulations pharmaceutiques du médicament de l'invention.

1/ Comprimés

Dérivé n° 1.......... 0,050 g

Excipient: lactose, sucre glace, amidon de rizi acide alginique, stéarate de magnésium.

2/ Comprimés dragéifiés

Dérivé n°10......... 0,100 g

Excipient: stéarate de magnésium, amidon de mais, gomme arabique, gomme laque, sucre blanc, glucose, cire blanche, cire de carnauba, paraffine, coccine nouvelle.

3/ Capsules

Dérivé n° 17......... 0,100 g

Excipient: stéarate de magnésium, amidon de mais, lactose.

4/ soluté injectable

Dérivé n° 4 0,075 g

solvant isotonique q.s.p. 3 ml

5/ Suppositoires

Dérivé n° 21......... 0,100 g

Triglycérides semi-synthétiques q.s.p. 1 suppositoire.

**Revendications** pour les Etats contractants BE CH DE FR GB IT LI LU NL SE

1 - Dérivés de la thiéno [3,2-c] pyridine de la formule générale I

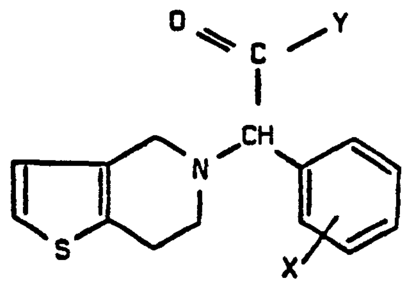

(I)

dans laquelle Y représente l'hydroxyle OH ou le groupe OR dans lequel R est un groupe alcoyle inférieur droit ou ramifié de 1 à 4 atomes de carbone, ou bien Y représente un groupe

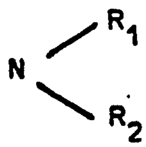

dans lequel $R_1$ et $R_2$ sont chacun indépendamment l'un de l'autre l'hydrogène ou un groupe alcoyle inférieur droit ou ramifié de 1 à 4 atomes de carbone, ou bien $R_1$ et $R_2$ forment ensemble et avec l'atome d'azote auquel ils sont rattachés, un groupe pyrrolidino, morpholino, pipéridino, ou benzyl-4 pipérazino; et X représente l'hydrogène, un halogène ou un radical alcoyle interieur de 1 à 4 atomes de carbone; et leurs sels d'addition avec les acides minéraux ou organiques pharmaceutiquement acceptables, lorsque Y représente les groupes OR ou

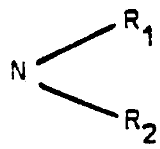

ou avec les bases minérales lorsque Y représente OH, ainsi que les deux énantiomères ou leur mélange.

2 - Procédé de préparation des composés selon la revendication 1, caractérisé en ce que l'on prépare les esters de formule générale I dans lesquels Y représente un groupe OR par condensation de la tétrahydro-4,5,6,7 thiéno [3,2-c] pyridine de formule II

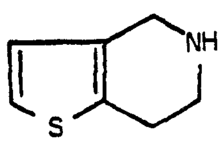

(II)

avec un α-chlorophénylacétate de formule générale III

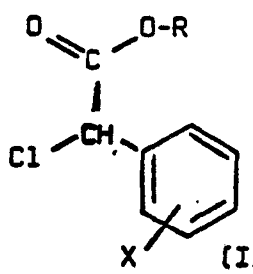

(III)

dans laquelle R et X prennent les valeurs définies ci-dessus, on obtient les acides de formule générale I dans laquelle Y représente OH, par saponification de ces esters, et ou peut transformer ces acides eventuellement après activation, en amide ou en ester de formule générale I par traitement avec une amine

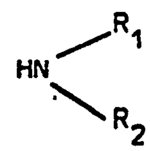

dans laquelle $R_1$ et $R_2$ prennent les valeurs définies ci-dessus ou avec un alcool R-OH dans lequel R a les significaticns précédentes.

3 - Procédé selon la revendication 2 caractérisé en ce que la réaction de condensation entre la thiéno-pyridine de formule II et l'ester de formule générale III s'effectue en présence d'un carbonate de métal alcalin, dans un solvant inerte, à des températures comprises entre 60° C et le point d'ébullition du solvant.

4 - Procédé selon la revendication 2 caractérisé en ce que la seponification de l'ester de formule générale I dans lequel R est méthyle ou éthyle est effectuée par un hydroxyde de métal alcalin, aus ein d'un solvant hydroalcoolique, à des températures comprises entre la température ambiante et le point d'ébullition du solvant.

5 - Procédé selon la revendicaticn 2 caractérisé en ce que l'activation de l'acide de formule générale I est obtenue par le chloroformiate d'éthyle en présence de triéthylamine à des températures comprises entre -5° C et 0° C dans un solvant inerte.

6 - Procédé selon la revendication 2 caractérisé en ce que l'activation de l'acide de formule générale I pour la préparation des amides a été effectuée par le dicyclohexylcarbodiimide.

7 - Procédé selon la revendication 2 caractérisé en ce que l'activation de l'acide de formule générale I pour la préparation des esters est effectuée par un courant de gaz chlorhydrique ou par le chlorure de thionyle.

8 - α-(tétrahydro-4,5,6,7 thiéno [3,2-c] pyridyl-5) o.chlorophénylacétate de méthyle

9 - α-(tétrahydro-4,5,6,7 thiéno [3,2-c] pyridyl-5) phénylacétate de méthyle

10 - α-(tétrahydro-4,5,6,7 thiéno [3,2-c] pyridyl-5) o.fluorophénylacétate de méthyle

11 - α-(tétrahydro-4,5,6,7 thiéno [3,2-c] pyridyl-5) o.chlorophénylcétate d'isopropyle

12 - α-(tétrahydro-4,5,6,7 thiéno [3,2-c] pyridyl-5) o.chlorophénylacétate d'éthyle

13 - N,N-diméthyl [α-(tétrahydro-4,5,6,7 thiéno [3,2-c] pyridyl-5] o.chlorophénylacétamide

14 - Médicament présentant en particulier des activités inhibitrice de l'agrégation plaquettaire et anti-thrombotique caractérisé en ce qu'il contient, à titre de principe actif, un dérivé de formule générale I suivant la revendication 1, ou un sel d'addition avec un acide minéral ou organique pharmaceutiquement acceptable de celui-ci ou avec les bases minérales, ainsi qu'un des deux énantiomères ou leur mélange.

15 - Médicament selon la revendication 14 caractérisé en ce qu'il est présenté sous forme de doses unitaires, le principe actif étant associé à un véhicule pharmaceutiquement approprié.

16 - Médicament selon l'une des revendications 14 ou 15 caractérisé en ce que chaque dose unitaire contient de 0,10 g à 1,00 g de principe actif.

**Revendications** pour l'Etat contractant AT

1- Procédé de préparation de dérivés de la thiénol [3,2-c] pyridine de formule générale I

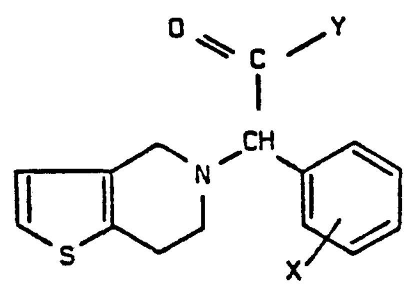

(I)

dans laquelle Y représente le groupe OH ou un groupe OR dans lequel R est un groupe alcoyle inférieur droit ou ramifie de 1 à 4 atomes de carbone ou bien Y représente un groupe

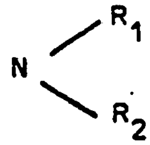

dans lequel $R_1$ et $R_2$ sont chacun indépendamment l'un de l'autre l'hydrogène ou un groupe alcoyle inférieur droit ou ramifié de 1 à 4 atomes de carbone, ou bien $R_1$ et $R_2$ forment ensemble et avec l'atome d'azote auquel ils sont rattachés, un groupe pyrrolidino, morpholino, pipéridino ou benzyl-4 pipérazono;

et x représente l'hydrogène, un halogène ou un radical alcoyle inférieur de 1 à 4 atomes de carbone; et de leurs sels d'addition avec les acides minéraux ou organiques pharmaceutiquement acceptables, lorsque Y représente les groupes OR ou

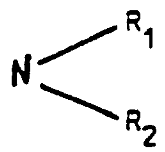

ou avec les bases minérales lorsque Y représente OH, ainsi que des deux énantioméres ou de leur mélange, caractérisé en ce que l'on prépare les esters de formule générale I dans lesquels Y représente un groupe OR par condensation de la tétrahydro-4,5,6,7 thiéno [3,2-c] pyridine de formule II

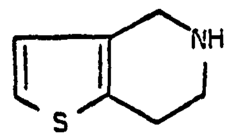

(II)

avec un α-chlorophénylacétate de formule générale III

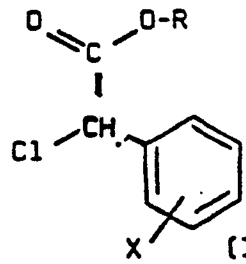

(III)

dans laquelle R et X ont les valeurs définies ci-dessus, on obtient les acides de formule générale I dans laquelle Y représente OH, par saponification de ces esters, et on peut transformer les acides, éventuellement après activation, en amide ou en ester de formule générale I par traitement avec une amine

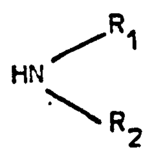

dans laquelle $R_1$ et $R_2$ ont les valeurs définies ci-dessus ou avec un alcool R-OH dans lequel R a les significations précédentes.

2. Procédé selon la revendication 1, caractérisé en ce que la réaction de condensation entre la thiéno-pyridine de formule II et l'ester de formule générale III s'effectue en présence d'un carbonate de métal alcalin, dans un solvant inerte, à des températures comprises entre 60°c et le point d'ébullition du solvant.

3. Procédé selon la revendication 1, caractérisé en ce que la saponification de l'ester de formule générale I dans lequel R est un groupe méthyle ou éthyle est effectuée par un hydroxyde de métal alcalin, au sein d'un solvant hydroalcoolique, à des températures comprises entre la température ambiante et le point d'ébullition du solvant.

4. Procédé selon la revendication 1, caractérisé en ce que l'activation de l'acide de formule générale I est obtenue par le chloroformiate d'éthyle en présence de triéthylamine à des températures comprises entre -5°C et 0°C dans un solvant inerte.

5. Procédé selon la revendication 1, caractérisé en ce que l'activation de l'acide de formule générale I pour la préparation des amides est effectuée par le dicyclohexylcarbodiimide.

6. Procédé selon la revendication 1, caractérisé en ce que l'activation de l'acide de formule générale I pour la préparation des esters est effectuée par un courant de gaz chlorhydrique ou par le chlorure de thionyle.

7. Procédé suivant la revendication 1, caractérisé en ce qu'on prépare le α-[tétrahydro-4,5,6,7 thiéno [3,2-c] pyridyl-5] o. chlorophényl-acétate de méthyle.

8.Procédé suivant la revendication 1, caractérisé en ce qu'on prépare le α-[tétrahydro-4,5,6,7 thiéno [3,2-c] pyridyl-5] phénylacétate de méthyle.

9. Procédé suivant la revendication 1 caractérisé en ce qu'on prépare le α-[tétrahydro-4,5,6,7 thiéno [3,2-c] pyridyl-5] o. fluoro phényl-acétate de méthyle.

10. Procédé suivant la revendication 1, caractérisé en ce qu'on prépare le α-[tétrahydro-4,5,6,7 thiéno [3,2-c] pyridyl-5] o.chloro phényl-acétate d'isopropyle.

11. Procédé suivant la revendication 1, caractérisé en ce qu'on prépare le α-[tétrahydro-4,5,6,7 thiéno [3,2-c] pyridyl-5] o. chloro phényl-acétate d'éthyle.

12. Procédé suivant la revendication 1, caractérisé en ce qu'on prépare le N,N-diméthyl [α-[tétrahydro-4, 5,6,7 thiéno [3,2-c] pyridyl-5] o. chloro phénylacétamide.

**Claims** for the contracting States: BE, CH, DE, FR, GB, IT, LU, NL, SE

1. Thieno (3 2-x) pyridine derivatives having the general formula:

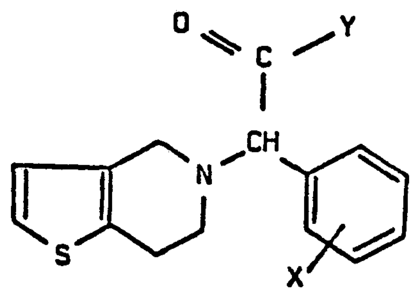

(I)

in which Y represents the OH group or an OR group in which R is a straight or branched lower alkyl radical of 1 to 4 carbon atoms, or Y represents a group

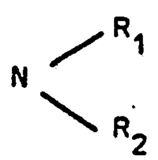

in which $R_1$ and $R_2$ are each independently of each other hydrogen or a straight or branched lower alkyl group of 1 to 4 carbon atoms; or $R_1$ and $R_2$ form together and with the nitrogen atom to which they are attached a pyrrolidino morpholino, piperidino or 4 benzye-piperazino group; and X represents hydrogen, a halogen or a lower alkyl radical of 1 to 4 carbon atoms; and their addition salts with pharmaceutically acceptable or organic acids if Y represents OR group or

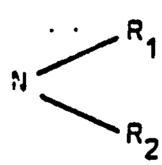

or with mineral bases if Y represents OH, as well as the two enantiomers or their mixture.

2. Process for the preparation of compounds as claimed in claim 1 wherein the esters of general formula (I), in which Y represents an OR group, are prepared by condensation of 4,5,6,7- tetrahydro-thieno (3,2-c) pyridine having formula II

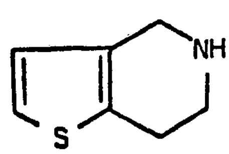

(II)

with an α-chloro phenylacetate having general formula III

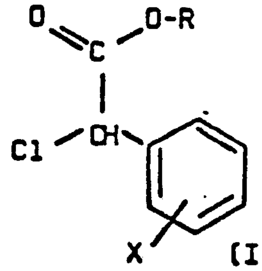

(III)

in which R and X have the above-defined meanings, the acids of general formula I in which Y represents OH are obtained by saponification of these esters and these acids may be converted optionally after activation, into an amide or an ester of general formula I by treatment with an amide

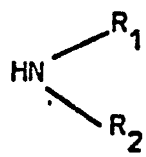

in which $R_1$ and $R_2$ are as defined above or with an alcohol R-OH in which R is as defined above.

3. Process as claimed in claim 2 wherein the condensation reaction between the thieno-pyridine of the formula II and the ester of the formula III is carried out in the presence of an alkali metal carbonate in an inert solvent, at temperatures between 60° C and the boiling point of the solvent.

4 - Process as claimed in claim 2 wherein the saponification of the ester of general formula I in which R is methyl or ethyl is carried out by an alkali metal hydroxide in a hydroalcoholic solvent at temperatures between room temperature and the boiling point of the solvent.

5. Process as claimed in claim 2 wherein the acid of general formula I is activated by ethyl chloroformate in the presence of triethylamine at temperatures between -5° C and 0° C in an inert solvent.

6.- Process as claimed in claim 2 wherein the acid of general formula I used for the preparation of the amides is activated with dicyclohexylcarbodiimide.

7 - Process as claimed in claim 2 wherein the acid of general formula I used for the preparation of the esters is activated by a stream of hydrochloric gas or by thionyl chloride.

8 - Methyl α-(4,5,6,7-tetrahydro-thieno (3,2-c)-5-pyridyl)-o. chlorophenyl-acetate.

9 - Methyl α-(4,6,6,7-tetrahydro-thieno (3,2-c)-5-(-pydidyl) phenylacetate.

10 - Methyl α-(4,5,6,7-tetrahydro-thieno (3,2-c)-5-pyridyl)-o. fluoro-phenylacetate.

11 - Isopropyl α-(4,5,6,7-tetrahydro-thieno (3,2-c)-5-pyridyl)-o. chloro-phenylacetate.

12 - Ethyl α-(4,5,6,7-tetrahydro-thieno (3,2-c)-5-pyridyl)-o. chloro-phenylacetate.

13 - N,N-dimethyl - (-α-(4,5,6,7-tetrahydro-thieno (3,2-c)-5-pyridyl)-o. chlorophenylacetamide.

14 - Medicament having in particular blood-platelet aggregation inhibiting activities and anti-thrombotic activities containing as active ingredient a derivative of general formula I as claimed in claim 1, or an addition salt thereof with a pharmaceutically acceptable mineral or organic acid or with mineral bases, as well as one of the two enantiomers of their mixture.

15 - Medicament as claimed in claim 14, in unit dosage form, the active ingredient being associated with a pharmaceutically acceptable carrier.

16 - Medicament as claimed in claim 14 or 15 in unit dosage form, each unit containing from 0.10 g to 1.00 g active ingredient.

**Claims** for the contracting State: AT

1. Process for the preparation of thieno (3,2-x) pyridine derivatives having the formula:

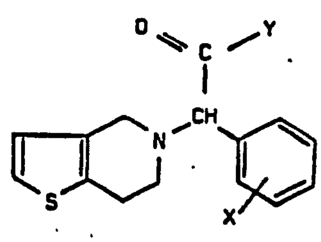

(I)

in which Y represents the OH group or an OR group in which R is a straight or branched lower alkyl radical, of 1 to 4 carbon atoms, or Y represents a group

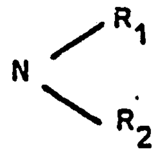

in which $R_1$ and $R_2$ are each independently of each other hydrogen or a straight or branched lower alkyl group of 1 to 4 carbon atoms; or $R_1$ and $R_2$ form together and with the nitrogen atom to which they are attached a pyrrolidino, morpholino, piperidino or 4-benzye-piperazino group; and X represents hydrogen, a halogen or a lower alkyl radical of 1 to 4 carbon atoms; and their addition salts with pharmaceutically acceptablé or organic acids if Y represents OR groups or

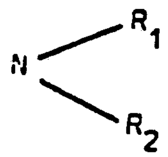

or with mineral bases if Y represents OH, as well as the two enantiomers or their mixture, wherein the esters of general formula I, in which Y represents an OR group, are prepared by condensation of 4,5,6,7-tetrahydro-thieno (3,2-c) pyridine having formula II

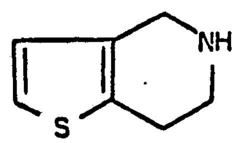 (II)

with an α -chlorophenylacetate having general formula III

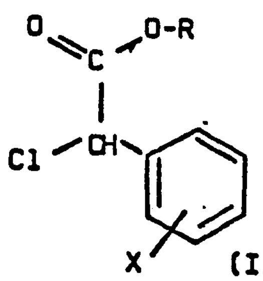

(III)

in which R and X have the above-defined meanings, the acids of general formula I in which Y represents OH are obtained by saponification of these esters, and these acids may be converted optionally after activation, into an amide or an ester of general formula I by treatment with an amine

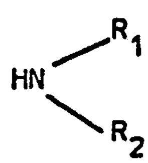

in wich $R_1$ and $R_2$ are as defined above or with an alcohol R-OH in which R is as defined above.

2. Process as claimed in claim 1 wherein the condensation reaction between the thieno-pyridine of the formula II and the ester of the formula III is carried out in the presence of an alkali metal carbonate in an inert solvent, at temperatures between 60° C and the boiling point of the solvent.

3. Process as claimed in claim 1 wherein the saponification of the ester of general formula I in which R is methyl or ethyl is carried out by an alkali metal hvdroxide in a hydroalcoholic solvent at temperatures between room temperature and the boiling point of the solvent.

4. Process as claimed in claim 1 wherein the acid of general formula I is activated by ethyl chloroformate in the presence of triethylamine at temperatures between -5° C and 0° C in an inert solvent.

5. Process as claimed in claim 1 wherein the acid of general formula I used for the preparation of the amides is activated with dicyclohexyl-carbodiimide.

6. Process as claimed in claim 1 wherein the acid of general formula I used for the preparation of the esters is activated by a stream of hydrochloric gas or by thionyl chloride.

7 - Process according to claim 1 wherein Methyl α-(4,5,6,7-tetrahydro-thieno (3 2-c)-5-pyridyl)-o. chlorophenyl-acetate is prepared.

8 - Process according to claim 1 wherein Methyl α-(4,5,6,7-tetrahydro-thieno (3,2-c)-5-pyridyl) phenylacetate is prepared.

9 - Process according to claim 1 wherein Methyl α-(4,5,6,7-tetrahydro-thieno (3,2-c)-5-pyridyl)-o. fluorophenylacetate is prepared.

10 - Process according to claim 1 wherein isopropyl α-(4,5, 6,7-tetrahydro-thieno (3,2-c)-5-pyridyl)-o. chlorophenylacetate is prepared.

11 - Process according to claim 1 wherein ethyl α-(4,5,6,7-tetrahydro-thieno (3,2-c)-5-piridyl)-o. chlorophenylacetate is prepared.

12 - Process according to claim 1 wherein N,N-dimethyl-(α-(4,5,6,7-tetrahydro-thieno (3,2-c)-5-pyridyl)-o. chlorophenylacetamide is prepared.

**Patentansprüche** für die Vertragsstaaten: BE, CH, DE, FR, GB, IT, LI,

LU, NL, SE

1. Derivate von Thieno[3,2-c]pyridin der allgemeinen Formel I

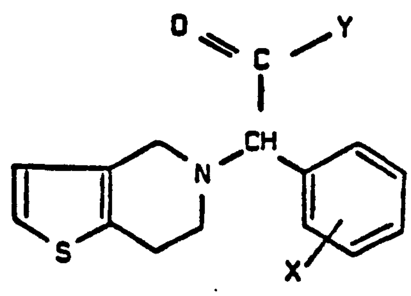

(I)

worin

Y das Hydroxyl OH oder die Gruppe OR, in der R ein gerader oder verzweigter niederer Alkylrest mit 1 bis 4 C-Atomen ist, oder worin Y auch eine Gruppe

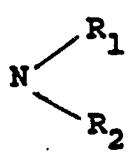

darstellt, in der

$R_1$ und $R_2$ jeweils unabhängig voneinander Wasserstoff oder ein gerader oder verzweigter niederer Alkylrest mit 1 bis 4 C-Atomen sind oder

$R_1$ und $R_2$ gemeinsam mit dem Stickstoffatom, an das sie gebunden sind, eine Pyrrolidino-, Morpholino-, Piperidino- oder 4-Benzylpiperazino-gruppe bilden, und

X Wasserstoff, ein Halogen oder einen niederen Alkylrest mit 1 bis 4 C-Atomen darstellt,

und ihre Additionssalze mit pharmazeutisch unbedenklichen anorganischen oder organischen Säuren, wenn Y die Gruppen OR oder

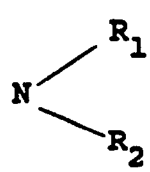

darstellt, oder mit anorganischen Basen, wenn Y CH darstellt, sowie die beiden Enantiomeren oder ihr Gemisch.

2. Verfahren zur Herstellung der Verbindungen nach Anspruch 1, dadurch gekennzeichnet, daß man die Ester der allgemeinen Formel I, in denen Y eine Gruppe OR ist, durch Kondensation von 4,5,6,7-Tetrahydrothieno-[3,2-c]pyridin der Formel II

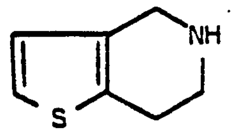

(II)

mit einem α-Chlorophenylacetat der allgemeinen Formel III

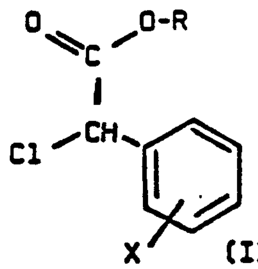

(III)

in der R und X die vorstehend genannten Werte annehmen, herstellt, die Säuren der allgemeinen Formel I, in der Y für OH steht, durch Verseifung dieser Ester erhält, und man diese Säuren gegebenenfalls nach Aktivierung in das Amid oder in den Ester der allgemeinen Formel I durch Behandlung mit einem Amin

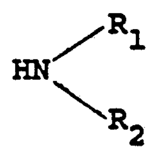

,

worin $R_1$ und $R_2$ die Vorstehend genannten Werte annehmen, oder mit einem Alkohol R-OH, worin R die vorstehend genannten Bedeutungen hat, umwandeln kann.

3. Verfahren nach Anspruch 2, dadurch gekennzeichnet, daß die Kondensationsreaktion zwischen dem Thienopyridin der Formel II und dem Ester der allgemeinen Formel III in Gegenwart eines Alkalimetallcarbonats in einem inerten Lösungsmittel bei Temperaturen, die zwischen 60°C und dem Siedepunkt des Lösungsmittels liegen, durchgeführt wird.

4. Verfahren nach Anspruch 2, dadurch gekennzeichnet, daß die Verseifung des Esters der allgemeinen Formel I, worin R Methyl oder Ethyl ist, mit einem Alkalimetallhydroxid innerhalb eines wässrig-alkoholischen Lösungsmittels bei Temperaturen, die zwischen Umgebungstemperatur und dem Siedepunkt des Lösungsmittels liegen, durchgeführt wird.

5. Verfahren nach Anspruch 2, dadurch gekennzeichnet, daß die Aktivierung der Säure der allgemeinen Formel I mit dem Ethylchloroformiat in Gegenwart von Triethylamin bei Temperaturen zwischen -5°C und 0°C in einem inerten Lösungsmittel erzielt wird.

6. Verfahren nach Anspruch 2, dadurch gekennzeichnet, daß die Aktivierung der Säure der allgemeinen Formel I für die Herstellung der Amide durch das Dicyclohexylcarbodiimid durchgeführt worden ist.

7. Verfahren nach Anspruch 2, dadurch gekennzeichnet, daß die Aktivierung der Säure der allgemeinen Formel I für die Herstellung der Ester mit einem Salzsäuregasstrom oder mit dem Thionylchlorid durchgeführt wird.

8. α-(4,5,6,7-Tetrahydrothieno[3,2-c]5-pyridyl)-o-chlorophenylessigsäuremethylester.

9. α-(4,5,6,7-Tetrahydrothieno[3,2-c]5-pyridyl)phenyl-essigsäuremethylester.

10. α-(4,5,6,7-Tetrahydrothieno[3,2-c]5-pyridyl)-o-fluorophenylessigsäuremethylester.

11. α-(4,5,6,7-Tetrahydrothieno[3,2-c]5-pyridyl)-o-chlorophenylessigsäureisopropylester.

12. α-(4,5,6,7-Tetrahydrothieno[3,2-c]5-pyridyl)-o-chlorophenylessigsäureethylester.

13. N,N-Dimethyl[α-(4,5,6,7-Tetrahydrothieno[3,2-c]5-pyridyl)-o-chlorophenylacetamid.

14. Medikament, das insbesondere hemmende Wirkungen auf die Agglomeration der Blutplättchen und antithrombotische Aktivität aufweist, dadurch gekennzeichnet, daß es als Wirkstoff ein Derivat der allgemeinen Formel I gemäß Anspruch 1 oder ein Additionssalz mit einer pharmazeutisch unbedenklichen anorganischen oder organischen Säure oder mit anorganischen Basen sowie eines der beiden Enantiomeren oder ihr Gemisch enthält.

15. Medikament nach Anspruch 14, dadurch gekennzeichnet, daß es in Form von Einheitsdosen dargeboten wird, wobei der Wirkstoff mit einem pharmazeutisch geeigneten Träger verbunden ist.

16. Medikament nach einem der Ansprüche 14 oder 15, dadurch gekennzeichnet, daß jede Einheitsdosis 0,10 g bis 1,00 g Wirkstoff enthält.

**Patentansprüche** für den Vertragsstaat: AT

1. Verfahren zur Herstellung von Derivaten von Thieno-[3,2-c]pyridin der allgemeinen Formel I

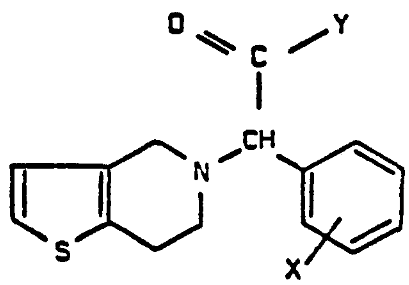

(I)

in der

Y die Gruppe OH oder eine Gruppe OR darstellt, in der R ein gerader oder verzweigter niederer Alkylrest mit 1 bis 4 C-Atomen ist, oder worin Y auch eine Gruppe

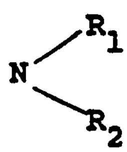

darstellt, in der

$R_1$ und $R_2$ jeweils unabhängig voneinander Wasserstoff oder ein gerader oder verzweigter niederer Alkylrest mit 1 bis 4 C-Atomen sind oder

$R_1$ und $R_2$ gemeinsam mit dem Stickstoffatom, an das sie gebunden sind, eine Pyrrolidino-, Morpholino-, piperidino- oder 4-Benzylpiperazino-gruppe sind, und

X Wasserstoff, ein Halogen oder einen niederen Alkylrest mit 1 bis 4 C-Atomen darstellt,

und ihre Additionssalze mit pharmazeutisch unbedenklichen anorganischen oder organischen Säuren, wenn Y die Gruppen OR oder $R_1$

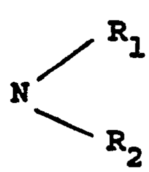

darstellt, oder mit anorganischen Basen, wenn Y OH darstellt, sowie der beiden Enantiomeren oder ihres Gemisches, dadurch gekennzeichnet, daß man die Ester der allgemeinen Formel I, in denen Y eine Gruppe OR darstellt, durch Kondensation von 4,5,6,7-Tetrahydro-thieno[3,2-c]pyridin der Formel II

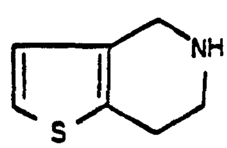

(II)

mit einem α-Chlorophenylacetat der allgemeinen Formel III

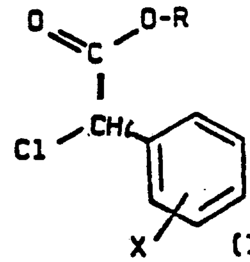

(III)

in der R und X die vorstehend genannten Werte haben, herstellt, man die Säuren der allgemeinen Formel I, in der Y für OH steht, durch Verseifung dieser Ester erhält und man diese Säuren gegebenenfalls nach Aktivierung zum Amid oder zum Ester der allgemeinen Formel I durch Behandlung mit einem Amin

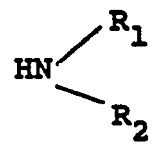 ,

in der $R_1$ und $R_2$ die vorstehend genannten Werte haben, oder mit einem Alkohol R-OH, worin R die vorstehend genannten Bedeutungen hat, umwandeln kann.

2. Verfahren nach Anspruch 1, dadurch gekennzeichnet, daß die Kondensationsreaktion zwischen dem Thienopyridin der Formel II und dem Ester der allgemeinen Formel III in Gegenwart eines Alkalimetallcarbonats in einem inerten Lösungsmittel bei Temperaturen zwischen 60°C und dem Siedepunkt des Lösungsmittels durchgeführt wird.

3. Verfahren nach Anspruch 1, dadurch gekennzeichnet, daß die Verseifung des Esters der allgemeinen Formel I, in dem R ein Methyl- oder Ethylrest ist, mit einem Alkalimetallhydroxid in einem wäßrig-alkoholischen Lösungsmittel bei Temperaturen zwischen der Umgebungstemperatur und dem Siedepunkt des Lösungsmittels durchgeführt wird.

4. Verfahren nach Anspruch 1, dadurch gekennzeichnet, daß die Aktivierung der Säure der allgemeinen Formel I mit Ethylchloroformiat in Gegenwart von Triethylamin bei Temperaturen zwischen -5°C und 0°C in einem inerten Lösungsmittel erzielt wird.

5. Verfahren nach Anspruch 1, dadurch gekennzeichnet, daß die Aktivierung der Säure der allgemeinen Formel I für die Herstellung der Amide mit Dicyclohexylcarbodiimid durchgeführt wird.

6. Verfahren nach Anspruch 1, dadurch gekennzeichnet, daß die Aktivierung der Säure der allgemeinen Formel I für die Herstellung der Ester mit einem Salzsäuregasstrom oder mit Thionylchlorid durchgeführt wird.

7. Verfahren nach Anspruch 1, dadurch gekennzeichnet, daß man den α-(4,5,6,7-Tetrahydrothieno[3,2-c]5-pyridyl)-o-chlorophenylessigsäuremethylester herstellt.

8. Verfahren nach Anspruch 1, dadurch gekennzeichnet, daß man den α-(4,5,6,7-Tetrahydrothieno[3,2-c]5-pyridyl)phenylessigsäuremethylester herstellt.

9. Verfahren nach Anspruch 1, dadurch gekennzeichnet, daß man den α-(4,5,6,7-Tetrahydrothieno[3,2-c]5-pyridyl)-o-fluorophenylessigsäuremethylester herstellt.

10. Verfahren nach Anspruch 1, dadurch gekennzeichnet, daß man den α-(4,5,6,7-Tetrahydrothieno[3,2-c]5-pyridyl)-o-chlorophenylessigsäureisopropylester herstellt.

11. Verfahren nach Anspruch 1, dadurch gekennzeichnet, daß man den α-(4,5,6,7-Tetrahydrothieno[3,2-c]5-pyridyl)-o-chlorophenylessigsäureethylester herstellt.

12. Verfahren nach Anspruch 1, dadurch gekennzeichnet, daß man das N,N-Dimethyl[α-(4,5,6,7-Tetrahydrothieno-[3,2-c]5-pyridyl)]-o-chlorophenylacetamid herstellt.